# EUROPEAN PATENT SPECIFICATION

(11) **EP 1 978 817 B1**
(45) Date of publication and mention of the grant of the patent: **04.10.2017**
(21) Application number: 07763083.8
(22) Date of filing: 02.02.2007
(51) Int. Cl.: A61K 8/44, A61K 8/67, A61K 8/73, A61K 8/02, A23G 3/36, A23G 4/06, A61K 8/24, A61K 8/36, A61K 8/362, A61K 8/365, A61K 8/60, A61K 8/88, A61Q 11/00

(54) **CALCIUM PHOSPHATE SALTS IN ORAL COMPOSITIONS SUITABLE AS A TOOTH REMINERALIZING AGENT**
CALCIUMPHOSPHAT-SALZE IN ORALEN ZUSAMMENSETZUNGEN ALS ZAHNMINERALISIERUNGSMITTEL
SELS DE PHOSPHATE DE CALCIUM DANS DES COMPOSITIONS ORALES APPROPRIÉES COMME AGENT REMINÉRALISANT DENTAIRE

(30) Priority: 03.02.2006 US 765289 P
(43) Date of publication of application: 15.10.2008
(73) Proprietor: WM. WRIGLEY JR. COMPANY, Chicago, IL 60611 (US)
(72) Inventor: HAAS, Michael, S., Chicago, Illinois 60622 (US); GREENBERG, Michael, J., Chicago, Illinois 60622 (US)
(74) Representative: Campbell, Neil Boyd
(86) International application number: PCT/US2007/061533
(87) International publication number: WO 2007/092763

(56) References cited:
- EP-A- 0 535 816
- EP-A- 1 566 166
- WO-A-2005/048965
- US-A- 2 470 906
- US-A- 5 427 768
- US-A- 5 958 380
- US-A1- 2002 156 130

## Description

The present invention relates to a chewing gum composition comprising a calcium phosphate salt, a first acid and a second acid, the acids maximize the release of calcium and phosphate ions from the oral composition over an extended period of time, to promote the precipitation of enamel-like crystals on the surfaces of the teeth.

### SUMMARY OF THE INVENTION

Briefly, therefore, the present invention is directed to a chewing gum composition comprising a calcium phosphate salt, a first acid having a solubility of at least 25 weight percent in water at 20 °C and a second acid having a solubility of less than 20 weight percent in water at 20 °C, wherein the ratio of said first acid and said second acid is greater than 1:1, and wherein the calcium phosphate salt is a combination of tetracalcium phosphate and anhydrous dicalcium phosphate of from 1:1 to 5:1, wherein upon placement of the composition in an oral cavity dissolution of the first acid begins prior to dissolution of the second acid.

The present invention is further directed to a chewing gum composition comprising a tetracalcium phosphate salt, a first acid having a solubility of at least 25 weight percent in water at 20 °C and a second acid having a solubility of less than 20 weight percent in water at 20°C, wherein the ratio of said first acid and said second acid is greater than 1:1, and wherein the calcium phosphate salt is a combination of tetracalcium phosphate and anhydrous dicalcium phosphate of from 1:1 to 5:1, wherein the first acid has a solubility in an oral cavity which is greater than the solubility of the second acid.

Additional features and advantages of the present invention are detailed elsewhere herein, or will be apparent therefrom.

US2002/0156130 describes dental compositions and methods for preventing dental plaque and carie formation. The compositions described comprise organic acids, especially acetic acid. A particular embodiment describes a chewing gum comprising acetic acid and ascorbic acid.

US2470906 describes dentrifices comprising ascorbic acid or analogues of ascorbic acid.

US5427768 describes a method of treating dental tissue in order to remineralize caries lesions, cavities and root erosions of teeth. The method involves applying to the teeth a supersaturated calcium phosphate solution.

EP1566166 describes non-aqueous oral compositions containing hydroxypropyl cellulose and at least one of glycerol, dicycerol, propylene glycol, 1,3-butylene glycol and polyethylene glycol.

EP0535816 describes abrasive dentrifice compositions containing an oxygen liberating whitening compound which comprises a combination of a dicalcium phosphate compound and a metal ion free peroxide compound.

### DETAILED DESCRIPTION OF THE EMBODIMENTS

In accordance with the present invention, and as further detailed herein below, it has been discovered that the use of a composition as defined herein, upon placement of this composition in an oral cavity (e.g., to masticate or consume the composition), the release and precipitation of calcium and phosphate ions present therein in the oral cavity, and thus also acts to promote the subsequent remineralization of teeth exposed thereto.

Without being held to any particular theory, it is generally believed that, upon initial placement (e.g., mastication or consumption) of the composition of the present invention in an oral cavity, a first acid, more soluble in the saliva of the oral cavity than a second acid, initially dissolves. The dissolution of this first acid acts to (i) lower the pH of the saliva in the oral cavity, to for example a pH of greater than about 3 and less than about 6, or greater than about 4 and less than about 5, as well as (ii) alter the environment within the oral cavity, to promote or stimulate saliva flow. The lower salivary pH and increased saliva flow, as well as the acidic nature of the composition itself (the composition generally has a pH of less than about 6, less than about 5 or even less than about 4), in turn act to promote dissolution of the calcium phosphate salts present in the composition, which are otherwise sparingly soluble, releasing calcium and phosphate ions into the saliva. As the saliva flow continues to increase, or at least remain at an elevated level, the concentration of salivary electrolytes, basic proteins and salivary bicarbonate present therein, also increase, or remain at elevated levels. These factors, as well as the presence of calcium salts released from the composition itself, act to buffer the pH of the saliva to a more neutral, or possibly a slightly basic, pH (e.g., a pH of greater than about 6 to less than
about 8, or greater than about 6.5 to less than about 7.5). The elevated pH (i.e., a neutral or slightly basic pH) causes the calcium phosphate salts present in solution (i.e., dissolved in the saliva) to precipitate on a surface of the teeth, or in a subsurface region thereof, enabling the mineralization or remineralization thereof.

Typically, the elevated pH may act to slow, and potentially stop, the release of calcium phosphate salts from the oral composition, due to their reduced solubility in the saliva. However, in accordance with the present invention, the pH of the oral composition remains acidic (e.g., less than about 6, less than about 5 or even less than about 4), thus enabling the continued or subsequent dissolution or release of a second, less soluble acid (in comparison to the solubility of the first acid). Dissolution of the second acid acts to ensure the pH of the composition itself remains acidic even though the saliva pH is about neutral, if not slightly basic, for an extended period of time (e.g., at least about 5 minutes, about 10 minutes, about 20 minutes, about 30 minutes or more). As a result, dissolution of the calcium phosphate salts within the composition, and thus the release of these salts therefrom, continues to occur for an extended period of time, thus extending the period of time during which mineralization or remineralization may occur.

### 1. Calcium Phosphate Salts

### A. Compositions

Compounds that release calcium and phosphate ions may be selected from a number of commercially available compounds, and other compounds that are recognized as food additives in other contexts. All such additives encompassed by the present invention are intended to be non-toxic. For the purpose of this invention, the term "non-toxic" is intended to conform with accepted and established definitions of safety, such as are described by the designation "generally accepted as safe" by the Food and Drug Administration. Also encompassed in this
definition are those compounds that have been added to food for some time and which are recognized as safe under conditions of their intended use. The additives of the invention, including calcium phosphate salts, or calcium salts and phosphate salts, are sufficiently non-toxic for oral use at the intended levels on a regular basis, and are preferably stable for the desired shelf-life.

The chewing gum composition of the present invention comprises a mixture of tetracalcium phosphate and anhydrous dicalcium phosphate of from 1:1 to 5:1.

It is to be noted that the particle size of the salts employed herein is generally optimized for the chewing gum composition in which it is contained. Typically, however, for chewing gum compositions in which a gritty or granular texture is not desirable, the particle size of the salts may be less than about 750 microns, and may be less than about 500 microns or even less than about 250 microns, the particle size being in the range of, for example, greater than about 5 microns and less than about 100 microns, or greater than about 10 microns and less than about 50 microns. In contrast, for chewing gum compositions in which a gritty, granular, or crunchy, texture is desirable, the particle size may typically be greater than about 750 microns, and may be greater than about 1000 microns or even about 1500 microns, the particle size being in the range of, for example, greater than about 1000 microns and less than about 2000 microns, or greater than about 1250 microns and less than about 1500 microns.

In general, the concentration of the calcium phosphate salts, present in the chewing gum composition is sufficient to effect mineralization and/or remineralization of the teeth; that is, the chewing gum composition typically has a "mineralizing-remineralizing amount" of the calcium phosphate salt, which is an amount sufficient to effect mineralization or remineralization of the surface or subsurface lesions in teeth and/or to effect mineralization of exposed dentinal tubules. Typically, however, the concentration of the calcium phosphate salts, present in the chewing gum composition of the present invention is at least about 0.5 weight percent, based on the total weight of the chewing gum composition. However, the oral composition may alternatively comprise at least about 1 weight percent, at least about 2 weight percent, at least about 4 weight percent, at least about 6 weight percent, at least about 8 weight percent, at least about 10 weight percent, at least about 15 weight percent or more. For example, the chewing gum composition may have a concentration of the calcium phosphate salts, that falls within the range of greater than about 0.5 weight percent and less than about 10 weight percent, or greater than about 1 weight percent and less than about 8 weight percent, or greater than about 2 weight percent and less than about 6 weight percent, based on the total weight of the composition.

In this regard it is to be noted that the concentration of the above-noted salts in the chewing gum composition of the present invention may additionally or alternatively be expressed in terms of the concentration of calcium and/or phosphate ions in an aqueous solution or in the oral cavity itself (e.g., the concentration in the saliva present therein). Accordingly, the concentration of the above-noted salts present in the chewing gum composition may be sufficient to achieve a concentration of calcium and/or phosphate ions present in an aqueous solution, or in the saliva present in an oral cavity of the user or consumer of the composition, of at least about 100 parts per million (ppm), at least about 500 ppm, at least about 1000 ppm, at least about 5000 ppm, at least about 10,000 ppm or even at least about 15,000 ppm, the concentration being in the range of, for example, greater than about 100 ppm and less than about 15,000 ppm, or greater than about 500 ppm and less than about 10,000 ppm, or greater than about 1000 ppm and less than about 5000 ppm.

As noted above, the chewing gum composition of the present invention comprises (i) tetracalcium phosphate (i.e., "TTCP" or [Ca4O(PO4)2]), which is a sparingly soluble calcium phosphate salt that has a relatively high solubility at physiological conditions in comparison to other calcium phosphate salts, and (ii) anhydrous dicalcium phosphate (i.e., "DCPA" or [CaHPO4]), which is an acidic, sparingly soluble calcium phosphate salt utilized in food and dentifrice manufacturing, in a ratio of from 1:1 to 1:5 (w/w). TTCP is commercially available, for example from Himed, Inc. DCPA is commercially available, for example from Rhodia.

The weight ratio thereof ranges from 1:1 to 5:1, or from about 2:1 to about 4:1. In still another embodiment, the weight ratio of TTCP to DCPA may be greater than about 2:1, and still more preferably is greater than about 3:1.

It is to be further noted that, in addition to the calcium phosphate salts, the chewing gum composition of the present invention may additionally comprise at least one water-soluble fluoride salt. Suitable water-soluble fluoride salts for use in the present invention include, for example, the alkali metal or ammonium fluorides such as sodium, potassium, lithium or ammonium fluoride; tin fluoride; indium fluoride; zirconium fluoride; copper fluoride; nickel fluoride; palladium fluoride; fluorozirconates such as sodium, potassium or ammonium fluorozirconate or tin fluorozirconate; fluorosilicates; fluoroborates; fluorostannites; and, fluorophosphates, such as sodium fluorophosphate, potassium fluorophosphate and ammonium fluorophosphate. In addition, organic fluorides, such as the known amine fluorides, may also be used in the oral products of this invention. In an embodiment, sodium fluoride is used in the oral composition.

It is to be still further noted that, in order to avoid fluorosis and other toxic effects, it is generally desirable to limit ingestion of fluoride to, on an average, an amount of less than about 0.2 mg of fluoride from all sources for a given day. In view thereof, the concentration of fluoride provided in the chewing gum composition of the present invention is preferably appropriately limited. For example, the concentration of fluoride ions in the chewing gum is preferably no more than about 100 ppm, and more preferably is no more than about 50 ppm, the concentration for example falling within the range of about 1 to about 25 ppm, or about 5 to about 20 ppm.

### B. Coatings

Optionally, the calcium phosphate salts employed in the present invention may be encapsulated or coated with a barrier layer, such as a moisture barrier layer, in order for example to limit, or substantially prevent, the salt from interacting with one or more of the combination of acids present in the composition. Physical modifications of the calcium phosphate salts by encapsulation with another substrate may increase or delay their release by modifying the solubility or dissolution rate of the salts. For example, in one particular embodiment, the calcium phosphate salts may be encapsulated or coated with magnesium stearate. In such an embodiment, coating or encapsulation may be achieved by, for example, mixing magnesium stearate with the salt or salt blend in essentially any amount sufficient to achieve the desired result. Typically, however, the amount of magnesium stearate present in the resulting magnesium mixture is at least about 0.5 weight percent, at least about 1 weight percent, at least about 2 weight percent, at least about 4 weight percent or more, the concentration for example falling within the range of between about 0.5 weight percent and about 3 weight percent, or between about 1 and about 2 weight percent, based on the total weight of the mixture.

Any standard technique which gives partial or full encapsulation of the salts can be used. These techniques include, but are not limited to, spray drying, spray chilling, fluid-bed coating, extrusion, coextrusion, inclusion, granulation, roll compaction and coacervation. These encapsulation techniques, which give partial encapsulation of the salts or full encapsulation of the salts, can be used individually or in any combination in a single step process or multiple step process. Generally, delayed release of the salts can be obtained using multistep processes like spray drying the salt, followed by fluid-bed coating of the resultant powder.

The encapsulation techniques of the calcium phosphate salts here described are standard coating techniques and generally give varying degrees of coating, from partial to full coating, depending on the coating composition used in the process. Also, the coating compositions may be susceptible to water permeation to various degrees. Generally, compositions that have high organic solubility, good film-forming properties and low water solubility give better delayed release of the salts. Such compositions include acrylic polymers and copolymers, carboxyvinyl polymer, polyamides, polystyrene, polyvinyl acetate, polyvinyl acetate phthalate, polyvinylpyrrolidone, and waxes. Although all of these materials may be used for encapsulation of the mineralizing salts, typically only food-grade materials are considered. Two standard food-grade coating materials that are good film formers but not water soluble are shellac and Zein. Others which are more water soluble, but good film formers, are materials like agar, alginates, a wide range of cellulose derivatives like ethyl cellulose, methyl cellulose, sodium hydroxymethyl cellulose, and hydroxypropylmethyl cellulose, dextrin, gelatin, and modified starches. These ingredients, which are generally approved for food use, give a fast release when used as an encapsulant for the salts. Other encapsulants like acacia or maltodextrin can also encapsulate the calcium phosphate, or calcium salts and/or phosphate salts, and give a fast release of them in various oral compositions.

The amount of coating or encapsulating material on the calcium phosphate salts also controls the length of time for its release from oral compositions. Generally, the higher the level of water-insoluble coating and the lower the amount of salt, the slower the release of the salt during mastication. Also, the higher the usage level of a water-soluble coating, the slower the release rate. In an embodiment, to obtain the desired salt release to blend with a gum's flavor release, the encapsulant is typically a minimum of about 10 weight percent of the coated mineralizing salt. Preferably, the encapsulant is a minimum of about 20 to about 50 weight percent of the coated salt. Depending on the coating material, a higher or lower amount of coating material may be needed to give the desired release of salt to balance with acid release.

Another method of giving a modified release of the mineralizing salts is agglomeration of the salts with an agglomerating agent which partially coats the calcium phosphate salts. This method includes the step of mixing the salt and an agglomerating agent with a small amount of water or solvent. The mixture is prepared in such a way as to have individual wet particles in contact with each other so that a partial coating can be applied. After the water or solvent is removed, the mixture is ground and used as a powdered, coated product.

Materials that can be used as the agglomerating agent are the same as those used in encapsulation procedures mentioned previously. However, since the coating is only a partial encapsulation, and the calcium phosphate salts are slightly water-soluble, some agglomerating agents are more effective in modifying the salt release than others. Some of the better agglomerating agents are the organic polymers like acrylic polymers and copolymers, polyvinyl acetate, polyvinylpyrrolidone, waxes, shellac, and Zein. Other agglomerating agents are not as effective in giving the salts a delayed release as are the polymers, waxes, shellac and Zein, but may actually give a faster release. Other agglomerating agents include, but are not limited to, agar, alginates, a wide range of cellulose derivatives like ethyl cellulose, methyl cellulose, sodium hydroxymethyl cellulose, hydroxypropylmethyl cellulose, dextrin, gelatin, modified starches, and vegetable gums like guar gum, locust bean gum, and carrageenan. Even though the agglomerated mineralizing salt is only partially coated, when the quantity of coating is increased compared to the quantity of salt, the release of the salt can be delayed for a longer time during mastication. In an embodiment, the level of coating used in the agglomerated product is a minimum of about 5 weight percent. Preferably the coating level is a minimum of about 15 weight percent, and more preferably about 20 weight percent. Depending on the agglomerating agent, a higher or lower amount of agent may be needed to give the desired release of salt to balance with the acid release.

The calcium phosphate salts may be coated in a two-step process or multiple step process. The salt may be encapsulated with any of the materials as described previously and then the encapsulated salt can be agglomerated as described previously to obtain an encapsulated/agglomerated/mineralizing/remineralizing product that could be used in an oral composition to give a delayed release of the salt over time.

In another embodiment of this invention, the calcium phosphate salts may be absorbed onto another component which is porous and become entrapped in the matrix of the porous component. Common materials used for absorbing the mineralizing/ remineralizing salts include, but are not limited to, silicas, silicates, pharmasorb clay, spongelike beads or microbeads, amorphous sugars like spray-dried dextrose, sucrose, alditols, amorphous carbonates and hydroxides, including aluminum and calcium lakes, vegetable gums and other spray dried materials. Insoluble materials will give the calcium phosphate salts a delayed release, while water-soluble materials will give the calcium phosphate salts a fast release from an oral composition.

Depending on the type of absorbent material and how it is prepared, the amount of mineralizing salt that can be loaded onto the absorbent will vary. Generally materials like polymers or spongelike beads or microbeads, amorphous sugars and alditols and amorphous carbonates and hydroxides absorb an amount equal to about 10% to about 40% of the weight of the absorbent. Other materials like silicas and pharmasorb clays may be able to absorb about 20% to about 80% of the weight of the absorbent.

The general procedure for absorbing the salt onto the absorbent may be characterized as follows: an absorbent, like fumed silica powder, can be mixed in a powder blender and an aqueous solution of the slightly water soluble salts can be sprayed onto the powder as mixing continues. The aqueous solution can be about 0.1 weight percent calcium phosphate salt solids and higher solid levels may be used if temperatures up to 90 °C. are used. Generally, water is the solvent, but other solvents like alcohol could also be used if approved for use in food. As the powder mixes, the liquid is sprayed onto the powder. Spraying is stopped before the mix becomes damp. The still free-flowing powder is removed from the mixer and dried to remove the water or other solvent, and then ground to a specific particle size.

After the salts are absorbed onto an absorbent or fixed onto an absorbent, the salt can be coated by encapsulation, either fully or partially, as described elsewhere herein. Alternatively, another form of encapsulation may be used, which is by entrapment of an ingredient by fiber extrusion or fiber spinning into a polymer. Polymers that can be used for extrusion are PVAC, hydroxypropyl cellulose, polyethylene and other types of plastic polymers. A process of encapsulation by fiber extrusion is disclosed in, for example, U.S. Pat. No. 4,978,537. The water insoluble polymer may be preblended with the calcium phosphate salts prior to fiber extrusion, or may be added after the polymer is melted. As the extrudate is extruded, it results in small fibers that are cooled and ground. This type of encapsulation/entrapment generally gives a very long, delayed release of an active ingredient.

In view of the foregoing, it is to be noted that the four primary methods to obtain a modified release of mineralizing agents are: (1) encapsulation (either fully or partially), (2) agglomeration, to give partial encapsulation, (3) fixation or absorption, which also gives partial encapsulation, and (4) entrapment into an extruded compound. These four methods, combined in any usable manner which physically modifies the release or dissolvability of the calcium phosphate salt, or calcium and/or phosphate salts, are included in this invention.

Other methods of treating the calcium phosphate salts to modify or physically isolate the salt from other ingredients may also have some effect on its release rate and stability. In an embodiment, the calcium phosphate, calcium or phosphate salts may be added to the liquid inside a liquid center gum product. The center fill of a gum product may comprise one or more carbohydrate syrups, glycerin, thickeners, flavors, acidulants, colors, sugars and sugar alcohols in conventional amounts. The ingredients are combined in a conventional manner. The salt is dissolved in the center-fill liquid and the amount of salt added to the center-fill liquid is typically about 2 ppm to about 500 ppm, by weight of the entire chewing gum formula. This method of using mineralizing/remineralizing calcium phosphate salts in chewing gum can allow for a smooth release rate, and can reduce or eliminate possible reaction of the salt with a particular acid to enhance release, improve remineralization, and yield improved shelf stability.

Another embodiment of isolating the calcium phosphate salts is to use them in the coating/panning of a pellet chewing gum. Pellet or ball gum is prepared as conventional chewing gum, but formed into pellets that are pillow shaped, or formed into balls. The pellets/balls can be then sugar coated or panned by conventional panning techniques to make a unique sugar coated pellet gum. The salts are very stable and slightly water soluble, and can be easily added to a hot sugar solution prepared for sugar panning. The salts can also be added as a powder blended with other powders often used in some types of conventional panning procedures. Using salts isolates them from other gum ingredients, in particular, acidic compounds, and modifies their release rate in a chewing gum composition.

Conventional panning procedures generally coat with sucrose, but recent advances in panning have allowed the use of other carbohydrate materials to be used in the place of sucrose. Some of these components include, but are not limited to, dextrose, maltose, palatinose, xylitol, lactitol, hydrogenated isomaltulose and other new alditols or a combination thereof. These materials may be blended with panning modifiers including, but not limited to, gum arabic, maltodextrins, corn syrup, gelatin, cellulose type materials like carboxymethyl cellulose or hydroxymethyl cellulose, starch and modified starches, vegetable gums like alginates, locust bean gum, guar gum, and gum tragacanth, insoluble carbonates like calcium carbonate or magnesium carbonate and talc. Antitack agents may also be added as panning modifiers which allow the use of a variety of carbohydrates and sugar alcohols to be used in the development of new panned or coated gum products. Flavors may also be added with the sugar coating and with the calcium phosphate salt, or calcium and/or phosphate salts, to yield unique product characteristics.

In yet another embodiment, another type of pan coating would also isolate the calcium phosphate salts from the confectionary ingredients. This technique is referred to as film coating and is more common in pharmaceuticals than in confectionaries, but the procedures are similar. In pan coating, a film like shellac, Zein, or cellulose-type material is applied onto a pellet-type product forming a thin film on the surface of the product. The film is applied by mixing the polymer, a plasticizer and a solvent (pigments are optional) and spraying the mixture onto the pellet surface. This is done in conventional type panning equipment, or in more advanced side-vented coating pans. When a solvent like alcohol is used, extra precautions are needed to prevent fires and explosions, and specialized equipment is used.

Some film polymers can use water as the solvent in film coating. Recent advances in polymer research and in film coating technology eliminate the problem associated with the use of solvents in coating. These advances make it possible to apply aqueous films to a pellet or chewing gum product. As calcium phosphate salt, or calcium and/phosphate salts, are slightly water soluble, they can be added to this aqueous film solution and applied with the film to a confectionary or chewing gum product. The aqueous film, or even the alcohol solvent film, in which the salt may be dispersed may also contain a flavor along with the polymer and plasticizer. By adding the salt to the polymer/plasticizer/solvent system, either as an emulsion or solution, the salt can be added with sweeteners, or flavors to balance taste. The calcium phosphate salt, or calcium and/or phosphate salts, can also be dissolved in the aqueous solvent and coated on the surface with the aqueous film. This will give a unique release of the salt to a film coated product, depending on how acids are incorporated into the oral composition.

### 2. Acid Combination

### A. Compositions

As previously noted, the chewing gum composition of the present invention comprises a combination or mixture of acids having differing solubilities, or solubility rates. The chewing gum composition comprises one or more acids that, upon introduction into the oral cavity of a consumer, rapidly dissolves or releases from the oral composition. The chewing gum composition additionally comprises one or more acids that, upon introduction into the oral cavity of a consumer, slowly dissolves or releases from the oral composition.

A "rapid release" acid of the present invention is characterized by its solubility in water, such an acid having a solubility of at least about 25 weight percent in water (i.e., at least 25 g in 100 ml of water at about 20 °C.), and preferably has a solubility of at least about 30 percent, at least about 35 percent, at least about 40 percent, or more (e.g., at least about 50 percent, at least about 75 percent, or even at least about 100 percent). Suitable "rapid release" acids may include, for example, citric acid (solubility about 60 percent), malic acid (solubility about 55 percent), tartaric acid (solubility about 140 percent), acetic acid, tannic acid, lactic acid, gluconic acid, ascorbic acid, succinic acid, their salts, and the like. In contrast, a "slow release" acid of the present invention has a solubility in water of less than about 20 percent (i.e., less than 20 g in 100 ml of water at 20 °C.), and preferably has a solubility of less than about 20 percent, less than about 15 percent, less than about 10 percent, or less (e.g., less than about 5 percent, less than about 3 percent, less than about 1 percent, or even less than about 0.5 percent). Suitable "slow release" acids may include, for example, adipic acid (solubility about 1.4 percent), fumaric acid (solubility about 0.6 percent), polyglutamic acid (solubility about 5 percent), polyaspartic acid (solubility about 5 percent), propionic acid, alginic acid, stearic acid, glutamic acid, aspartic acid, fatty acids (such as oleic, linoleic, palmitic, lauric, capric, caprylic) their salts and the like. For example, in certain embodiments, the rapid release (i.e., first) acid is citric acid and the slow release (i.e., second) acid is fumaric acid.

In this regard it is to be further noted, however, that suitable acids for use in the present invention may be alternatively characterized in terms of the hydrophobicity of the acids. Hydrophobicity may be assessed for a given acid in terms of the logarithm of the octanol/water partition coefficient (i.e., Log Pow). The octanol/water partition coefficient of some potentially useful acids are provided below:

| **Acid** | **Log P_{ow}** |
|---|---|
| Citric | - 1.70 |
| Malic | - 1.26 |
| Tartaric | - 0.76 |
| Adipic | 0.08 |
| Fumaric | 0.25 |

Accordingly, the chewing gum composition of the present invention may comprise a combination of acids, wherein: (i) one of the acids has a negative Log Pow value, the value for example being less than about 0, less than about -0.5, less than about -1, less than about -1.5, or even less than about -2, and (ii) one of the acids has a positive Log Pow value, the value for example being greater than about 0, greater than about 0.1, greater than about 0.5, greater than about 1 or more.

It is to be still further noted that suitable acids for use in the present invention may be additionally characterized in terms of the pH they create in the saliva of the oral cavity and/or the oral composition itself when placed in the oral cavity. For example, the oral composition of the present invention may typically comprise a rapid releasing acid that is effective to reduce the pH of the saliva present in the oral cavity, and/or the initial pH of the oral composition itself, to a value in the range of greater than about 3 and less than about 6, or optionally greater than about 4 and less than about 5, within a short period of time after being placed in the oral cavity (e.g., within about 60 seconds, within about 45 seconds, within about 30 seconds, or even within about 15 seconds, after being placed in the oral cavity). In addition, the oral composition of the present invention may comprise a slow releasing acid that acts to ensure the pH of the oral composition remains acidic (i.e., within the range of about 3 to about 6, or about 4 to about 5) for a period of time sufficient to ensure the prolonged release of the calcium phosphate salts, present therein (e.g., for a period of at least about 5 minutes, at least about 10 minutes, at least about 20 minutes, at least about 30 minutes or more).

In general, the total acid concentration for a given chewing gum composition of the present invention is controlled in order to achieve the desired balance between the above-noted pH considerations, mineralization or remineralization of the teeth, and other common factors (e.g., taste or tartness, flavor, texture or "mouthfeel," etc.). Typically, however, total acid content is less than about 10 weight percent, less than about 5 weight percent, the concentration being for example about 4 weight percent, about 3 weight percent, about 2 weight percent, about 1 weight percent, or even about 0.5 weight percent, based on the total weight of the composition. Accordingly, the total acid concentration may typically fall within the range of less than about 10 weight percent or 5 weight percent and greater than about 0.5 weight percent, or less than about 4 weight percent and greater than about 1 weight percent, or less than about 3 weight percent and greater than about 2 weight percent. In yet another embodiment, however, the total concentration of acid in the chewing gum composition will fall within the range of at least about 2 weight percent and less than about 5 weight percent, and more preferably at least about 3 weight percent and less than about 4 weight percent.

The concentration of each individual acid present in the composition may be controlled in view of the determined total acid concentration, as detailed above. Additionally, the concentration of the rapid releasing and slow releasing acids, as well as the ratio between these two, may be controlled in order to optimize the rate or duration of release of the calcium phosphate salt, or calcium salt and phosphates salt, present in the oral composition. Typically, however, the concentration of each individual acid will be less than about 4 weight percent, the concentration being for example about 3 weight percent, about 2 weight percent, about 1 weight percent, or even about 0.5 weight percent, based on the total weight of the composition. Accordingly, the concentration of each acid may typically fall within the range of less than about 4 weight percent and greater than about 0.5 weight percent, or less than about 3 weight percent and greater than about 1 weight percent. In still another embodiment, however, the concentration of each acid in the chewing gum composition will fall within the range of at least about 0.5 weight percent and less than about 3 weight percent, and more preferably at least about 1 weight percent and less than about 2.5 weight percent.

With respect to the weight ratio of the fast releasing acid "first acid" (e.g., citric acid, malic acid, tartaric acid) to the slow releasing acid "second acid" (e.g., adipic acid, fumaric acid, polyglutamic acid, polyaspartic acid), it is to be noted that this ratio is greater than 1:1; that is, in another embodiment the weight of the fast releasing acid is in excess, relative to the weight of the slow release acid. More preferably, this ratio is greater than about 1.5:1, greater than about 2:1, greater than about 2.5:1 or more. Combinations or mixtures of acids include, for example, citric and fumaric acid, the weight ratio being for example about 2:1, or about 2.5:1. This ratio is at most 5:1.

It is to be further noted that the acid combinations or mixtures present in the chewing gum composition may preferably be buffered in the composition, by including for example an alkali metal salt therein. More preferably, the acid blend in the chewing gum composition is buffered with sodium or calcium or potassium citrate or tartarate, calcium or potassium phosphate, calcium gluconate, sodium acetate, sodium acid pyrophosphate, and sodium potassium tartarate and the like, alone or in any combination.

As detailed elsewhere herein, a portion of substantially all of one or more of the acids present in the chewing gum composition of the present invention may be encapsulated, in order for example to substantially limit, if not prevent, the exposure of acid-sensitive components present in the chewing gum composition thereto. In doing so, the premature degradation of these acid-sensitive components may in turn be substantially limited, if not prevented.

### B. Coatings

Physical modifications of the slow and fast release acids by encapsulation with another substrate will increase or delay their release by modifying the solubility or dissolution rate of the acids. Any standard technique which gives partial or full encapsulation of the acids can be used. These techniques include, but are not limited to, spray drying, spray chilling, fluid-bed coating, extrusion, coextrusion, inclusion, granulation, roll compaction and coacervation. These encapsulation techniques that give partial encapsulation of the acids, or full encapsulation of the acids, can be used individually or in any combination in a single step process or multiple step process. Generally, delayed release of the acids can be obtained using multi-step processes, like spray drying the acid, followed by fluid-bed coating of the resultant powder.

The encapsulation techniques of the slow and fast release acids described herein are standard coating techniques and generally give varying degrees of coating, from partial to full coating, depending on the coating composition used in the process. Also, the coating compositions may be susceptible to water permeation to various degrees. Generally, compositions that have high organic solubility, good film-forming properties and low water solubility give better delayed release of the acids. Such compositions include acrylic polymers and copolymers, carboxyvinyl polymer, polyamides, polystyrene, polyvinyl acetate, polyvinyl acetate phthalate, polyvinylpyrrolidone, and waxes. Although all of these materials are possible for encapsulation of the acids, typically only food-grade materials are considered. Two standard food-grade coating materials that are good film formers but not water soluble are shellac and Zein. Others which are more water soluble, but also good film formers, are materials like agar, alginates, a wide range of cellulose derivatives like ethyl cellulose, methyl cellulose, sodium hydroxymethyl cellulose, and hydroxypropylmethyl cellulose, dextrin, gelatin, and modified starches. These ingredients, which are generally approved for food use, give a fast release when used as an encapsulant for the acids. Other encapsulants, like acacia or maltodextrin, can also encapsulate the slow and fast release acids, and give a controlled release of them in various oral compositions in conjunction with mineralizaing/remineralizing agents.

The amount of coating or encapsulating material on the slow and fast release acids also controls the length of time for their release from the oral compositions. Generally, the higher the level of water-insoluble coating and the lower the amount of acid, the slower the release of the acid during mastication. Also, the higher the usage level of a water-soluble coating, the slower the release rate. In an embodiment, to obtain the desired salt release to blend with a gum's flavor release, the encapsulant is typically a minimum of about 10 weight percent of the coated acid. Preferably, the encapsulant is a minimum of about 20 to about 50 weight percent of the coated acid. Depending on the coating material, a higher or lower amount of coating material may be needed to give the desired release of acid to balance with the release of calcium phosphate salt, or calcium and/or phosphate salts, of the present invention.

Another method of giving a modified release of the slow and fast release acids is agglomeration of the acids with an agglomerating agent which partially coats the acid. This method includes the step of mixing the acid and an agglomerating agent with a small amount of water or solvent. The mixture is prepared in such a way as to have individual wet particles in contact with each other so that a partial coating can be applied. After the water or solvent is removed, the mixture is ground and used as a powdered, coated product.

Materials that can be used as the agglomerating agent are the same as those used in encapsulation procedures mentioned previously. However, since the coating is only a partial encapsulation, and in particular, the slow dissolving acids are slightly water-soluble, some agglomerating agents are more effective in modifying the acid release than others. This also applies to the fast release acids; some of the agglomerating agents are more effective in modifying their release than others. Some of the better agglomerating agents are the organic polymers like acrylic polymers and copolymers, polyvinyl acetate, polyvinylpyrrolidone, waxes, shellac, and Zein. Other agglomerating agents are typically not as effective in giving the acid a delayed release as are the polymers, waxes, shellac and Zein, but may actually give a faster release. Other agglomerating agents include, but are not limited to, agar, alginates, a wide range of cellulose derivatives like ethyl cellulose, methyl cellulose, sodium hydroxymethyl cellulose, hydroxypropylmethyl cellulose, dextrin, gelatin, modified starches, and vegetable gums like guar gum, locust bean gum, and carrageenan. In at least one embodiment, the agglomerated slow release acid is only partially coated, when the quantity of coating is increased compared to the quantity of acid, the release of the acid can be delayed for a longer time during mastication in an oral composition. In other embodiments, the level of coating used in the agglomerated product is a minimum of about 5 weight percent of the agglomerated product. Preferably the coating level is a minimum of about 15 weight percent, and more preferably about 20 weight percent, of the agglomerated product. Depending on the agglomerating agent, a higher or lower amount of agent may be needed to give the desired release of the slow or fast release acid to balance acid release with the calcium phosphate salt, or calcium and/or phosphate salts, in an oral composition.

The acids may be coated in a two-step process or multiple step process. The acids may be encapsulated with any of the materials as described previously, and then the encapsulated acid can be agglomerated as described previously to obtain an encapsulated/agglomerated/mineralizing/remineralizing product that could be used in an oral composition to give a delayed release of both the slow or fast release acids over time.

In another embodiment of this invention, the acids employed may be absorbed onto another component which is porous and become entrapped in the matrix of the porous component. Common materials used for absorbing the acid include, but are not limited to, silicas, silicates, pharmasorb clay, spongelike beads or microbeads, amorphous sugars like spray-dried dextrose, sucrose, alditols, amorphous carbonates and hydroxides, including aluminum and calcium lakes, vegetable gums and other spray dried materials. Insoluble materials will give the slow or fast dissolving acid a delayed release, while water-soluble materials will give the slow or fast dissolving acid a fast release from an oral composition.

Depending on the type of absorbent material and how it is prepared, the amount of acid that can be loaded onto the absorbent will vary. Generally materials like polymers or spongelike beads or microbeads, amorphous sugars and alditols and amorphous carbonates and hydroxides absorb an amount equal to about 10% to about 40% of the weight of the absorbent. Other materials, like silicas and pharmasorb clays, may be able to absorb about 20% to about 80% of the weight of the absorbent.

The general procedure for absorbing the acid onto the absorbent may be characterized as follows: an absorbent, like fumed silica powder, can be mixed in a powder blender and an aqueous solution of the acid can be sprayed onto the powder as mixing continues. The aqueous solution can be about 0.1 weight percent acid solids, and higher solid levels may be used if temperatures up to 90°C are used. Generally, water is the solvent, but other solvents like alcohol could also be used if approved for use in food. As the powder mixes, the liquid is sprayed onto the powder. Spraying is stopped before the mix becomes damp. The still free-flowing powder is removed from the mixer and dried to remove the water or other solvent, and then ground to a specific particle size.

After the acid is absorbed onto an absorbent or fixed onto an absorbent, the acid can be coated by encapsulation, as described elsewhere herein. Alternatively, another form of encapsulation may be used, which is entrapment of an ingredient by fiber extrusion or fiber spinning into a polymer. Polymers that can be used for extrusion are PVAC, hydroxypropyl cellulose, polyethylene and other types of plastic polymers. A process of encapsulation by fiber extrusion is disclosed in, for example, U.S. Pat. No. 4,978,537, which is hereby incorporated by reference. The water insoluble polymer may be preblended with the slow or fast dissolving acid prior to fiber extrusion, or may be added after the polymer is melted. As the extrudate is extruded, it results in small fibers that are cooled and ground. This type of encapsulation/entrapment generally gives a very long, delayed release of an active ingredient.

In view of the foregoing, it is to be noted that the four primary methods to obtain a modified release of an acid are: (1) encapsulation by spray drying (either fully or partially), (2) agglomeration to give partial encapsulation, (3) fixation or absorption which also gives partial encapsulation, and (4) entrapment into an extruded compound. These four methods, combined in any usable manner which physically modifies the release or dissolvability of the slow or fast dissolving acids, are included in this invention.

Other methods of treating the acids to modify or physically isolate the acids from other ingredients may also have some effect on its release rate and stability. In an embodiment, the acid (e.g., citric acid) may be added to the liquid inside a liquid center gum product. The center fill of a gum product may comprise one or more carbohydrate syrups, glycerin, thickeners, flavors, colors, sugars and sugar alcohols in conventional amounts. The ingredients are combined in a conventional manner. The acid (e.g., citric acid) is dissolved in the center-fill liquid and the amount of salt added to the center-fill liquid is about 2 ppm to about 500 ppm by weight of the entire chewing gum formula. This method of using an encapsulated acid (e.g., citric acid) in chewing gum can allow for a smooth release rate, aid in the release of the mineralizing/remineralizing salt employed, and can reduce or eliminate possible reaction of the acid with a particular mineralizing salt to enhance release and yield improved shelf stability.

Another embodiment of isolating the acids is to use them in the coating/panning of a pellet chewing gum. Pellet or ball gum is prepared as conventional chewing gum, but formed into pellets that are pillow shaped, or into balls. The pellets/balls can be then sugar coated or panned by conventional panning techniques to make a unique sugar coated pellet gum. The slightly water soluble acids can be easily added to a hot sugar solution prepared for sugar panning. The acids can also be added as a powder blended with other powders often used in some types of conventional panning procedures. Using this method isolates the acids from other gum ingredients, in particular, mineralizing/remineralizing salt compounds, and modifies their release rate in a chewing gum composition.

Conventional panning procedures generally coat with sucrose, but recent advances in panning have allowed the use of other carbohydrate materials to be used in the place of sucrose. Some of these components include, but are not limited to, dextrose, maltose, palatinose, xylitol, lactitol, hydrogenated isomaltulose and other new alditols or a combination thereof. These materials may be blended with panning modifiers including, but not limited to, gum arabic, maltodextrins, corn syrup, gelatin, cellulose type materials like carboxymethyl cellulose or hydroxymethyl cellulose, starch and modified starches, vegetable gums like alginates, locust bean gum, guar gum, and gum tragacanth, insoluble carbonates like calcium carbonate or magnesium carbonate and talc. Antitack agents may also be added as panning modifiers which allow the use of a variety of carbohydrates and sugar alcohols to be used in the development of new panned or coated gum products. Flavors may also be added with the acid coating to yield unique product characteristics.

In yet another embodiment, another type of pan coating would also isolate the acidic compounds from other confectionary ingredients. This technique is referred to as film coating and is more common in pharmaceuticals than in confectionaries, but the procedures are similar. A film-like shellac, Zein, or cellulose-type material is applied onto a pellet-type product forming a thin film on the surface of the product. The film is applied by mixing the polymer, a plasticizer and a solvent (pigments are optional), and spraying the mixture onto the pellet surface. This is done in conventional type panning equipment, or in more advanced side-vented coating pans. When a solvent like alcohol is used, extra precautions are needed to prevent fires and explosions, and specialized equipment is used.

Some film polymers can use water as the solvent in film coating. Recent advances in polymer research and in film coating technology eliminates the problem associated with the use of solvents in coating. These advances make it possible to apply aqueous films to a candy, lozenge, pressed tablet or chewing gum product. As some acids are very water soluble and other acids are only slightly water soluble, they can be added to this aqueous film solution and applied with the film to a confectionary or chewing gum product. The aqueous film or even the alcohol solvent film, in which the acid may be dispersed, may also contain a flavor along with the polymer and plasticizer. By adding the acid to the polymer/plasticizer/solvent system, either as an emulsion or solution, the acid can be added with sweeteners, or flavors to balance taste. The acids can also be dissolved in the aqueous solvent and coated on the surface with the aqueous film. This will give a unique release of the acid to a film coated product, depending on how acids are incorporated into the oral composition.

### 3. Oral Composition

The present invention is directed to chewing gums (e.g., tablet gums, pellet or dragee gums, stick gums, compressed gums, co-extruded layered gums, bubble gum, etc.).

### A. Chewing Gums

The oral composition of the present invention is a chewing gum. In at least one embodiment, and as further detailed elsewhere herein, the chewing gum composition is a co-extruded layered gum, wherein for example the gum comprises one layer which comprises a buffered acid mixture or combination, and either: (i) one layer which comprises the calcium phosphate salts; or, (ii) two layers which comprise a calcium phosphate salts, wherein the layer comprising the buffered acid mixture is present (i.e., sandwiched) therebetween. In this way, the calcium phosphate salts, as well as the combination or acids, are essentially encapsulated in the gum base, and thus premature contact of these is limited, and preferably is substantially prevented.

In another embodiment, chewing gums are utilized for delivering the mineralizing or remineralizing components of the present invention because the inherent nature of chewing gum allows prolonged contact with the teeth. Furthermore, the base of the gum may provide sustained release of these components, thus minimizing the amount of these components that are used. The chewing gums of the present invention may be prepared using methods known in the art, including the methods described, for example, in U.S. Pat. No. 6,627,234.

In general, a chewing gum composition typically comprises a water-insoluble base portion, a water-soluble bulk portion, and typically flavoring agents. The water-soluble bulk portion dissipates with a portion of the flavoring agent over a period of time during chewing. The gum base portion is retained in the mouth throughout the chew. Generally, the moisture content of chewing gums of the present invention is less than about 5 weight percent, or less than about 2 weight percent.

The chewing gum may typically comprise about 5 percent to about 95 percent of the insoluble gum base, by weight of the chewing gum, but more commonly constitutes about 10 percent to about 50 percent of the gum base. The insoluble gum base may generally comprise one or more of the following: elastomers, resins, fats and/or oils, softeners, inorganic fillers, and/or waxes. For example, the gum base may comprise about 20 to about 60 weight percent of a synthetic elastomer, up to about 30 weight percent of a natural elastomer, about 5 to about 55 weight percent of an elastomer plasticizer, about 5 to about 35 weight percent of a filler, about 5 to about 35 weight percent of a softener, and optionally other minor amounts (e.g., about 1 weight percent or less) of miscellaneous ingredients such as colorants, antioxidants, etc., all based on the total weight of the gum base.

Synthetic elastomers may include, but are not limited to, polyisobutylene (having for example a weight average molecular weight of about 10,000 to about 95,000), isobutylene-isoprene copolymer (butyl elastomer), styrene copolymers (having for example a styrene-butadiene ratio of about 1:3 to about 3:1), polyvinyl acetate (having for example a weight average molecular weight of about 2,000 to about 90,000), polyisoprene, polyethylene, vinyl acetate-vinyl laurate copolymer (having for example a vinyl laurate content of about 5% to about 50% by weight of the copolymer), and combinations thereof.

Natural elastomers may include for example natural rubbers, such as smoked or liquid latex and guayule, as well as natural gums such as jelutong, lechi caspi, perillo, sorva, massaranduba balata, massaranduba chocolate, nispero, rosindinha, chicle, gutta hang kang, and combinations thereof. The preferred synthetic elastomer and natural elastomer concentrations vary depending on, for example, whether the chewing gum in which the base is used is adhesive or conventional, bubble gum or regular gum.

Elastomer plasticizers may include, but are not limited to, natural rosin esters such as glycerol esters or partially hydrogenated rosin, glycerol esters of polymerized rosin, glycerol esters of partially dimerized rosin, glycerol esters of rosin, pentaerythritol esters of partially hydrogenated rosin, methyl and partially hydrogenated methyl esters of rosin, pentaerythritol esters of rosin, synthetics such as terpene resins, and/or any suitable combinations of the foregoing. The preferred elastomer plasticizers will also vary depending on the specific application, and on the type of elastomer which is used.

Fillers or texturizers may include for example magnesium and calcium carbonate, ground limestone, silicate types such as magnesium and aluminum silicate, clay, alumina, talc, titanium oxide, mono-, di- and tri-phosphate, cellulose polymers, such as wood, and combinations thereof. Softeners or emulsifiers may include for example tallow, hydrogenated tallow, hydrogenated and partially hydrogenated vegetable oils, cocoa butter, glycerol monostearate, glycerol triacetate, lecithin, mono and triglycerides, acetylated monoglycerides, fatty acids (e.g., stearic, palmitic, oleic and linoleic acids), and combinations thereof. Colorants and whiteners may include FD&C dyes and lakes, fruit and vegetable extracts, titanium dioxide, and combinations thereof.

In addition to a water-insoluble gum base portion, a typical chewing gum composition includes a water-soluble bulk portion and one or more flavoring agents. The water-soluble portion can include bulk sweeteners, high intensity sweeteners, flavoring agents, softeners, emulsifiers, colors, acidulants, fillers, antioxidants, and other components that provide desired attributes.

Softeners may be added to the chewing gum in order to optimize chewability and/or mouthfeel of the gum. The softeners, which may also be known as plasticizers or plasticizing agents, typically constitute between about 0.5 to about 15 weight percent of the chewing gum. The softeners may include, for example, glycerin, lecithin, and combinations thereof. Aqueous sweetener solutions, such as those containing sorbitol, hydrogenated starch hydrolysates, corn syrup and combinations thereof, may also be used as softeners and binding agents in chewing gum.

Bulk sweeteners include both sugar and sugarless components. Bulk sweeteners typically constitute about 5 to about 95 weight percent of the chewing gum, but more typically constitute about 20 to about 80 weight percent, or about 30 to about 60 weight percent, of the gum. Sugar sweeteners generally include saccharide components commonly known in the chewing gum art, including but not limited to, sucrose, dextrose, maltose, dextrin, dried invert sugar, fructose, levulose, galactose, corn syrup solids, and the like, alone or in combination. Sugarless sweeteners include, but are not limited to, tagatose, trehalose, sugar alcohols (such as sorbitol, mannitol, xylitol, erythritol and isomalt), hydrogenated starch hydrolysates, maltitol, and the like, alone or in combination.

High intensity artificial sweeteners can also be used, alone or in combination, with the above. Exemplary sweeteners include, but are not limited to, sucralose, aspartame, NAPM derivatives such as neotame, salts of acesulfame, altitame, saccharin and its salts, cyclamic acid and its salts, glycyrrhizinate, dihydrochalcones, thaumatin, monellin, stevioside, and the like, alone or in combination. In order to provide longer lasting sweetness and flavor perception, it may be desirable to encapsulate or otherwise control the release of at least a portion of the artificial sweetener, using techniques generally known in the art to achieve the desired release characteristics. Such known techniques include, for example, wet granulation, wax granulation, spray drying, spray chilling, fluid bed coating, coacervation, and fiber extension.

Combinations of sugar and/or sugarless sweeteners may be used in the chewing gum. Additionally, the softener may also provide additional sweetness, such as with aqueous sugar or alditol solutions.

If a low calorie gum is desired, a low caloric bulking agent can be used. Examples of low caloric bulking agents include for example polydextrose, raftilose, raftilin, fructooligosaccharides (NutraFlora), palatinose oligosaccharide, guar gum hydrolysate (Sun Fiber), or indigestible dextrin (Fibersol). However, other low calorie bulking agents can be used.

A variety of flavoring agents, in varying amounts, can also be used if desired. Typically, the flavoring agent may be used in amounts of about 0.1 to about 15 weight percent of the gum, or about 0.2 to about 5 weight percent. Flavoring agents may include, for example, essential oils, synthetic flavors or mixtures thereof including, but not limited to, oils derived from plants and fruits such as citrus oils, fruit essences, peppermint oil, spearmint oil, other mint oils, clove oil, oil of wintergreen, anise and the like. Artificial flavoring agents and components may also be used, and natural and artificial flavoring agents may be combined in any acceptable fashion. The flavoring agent may also include a cooling agent to enhance the flavor and perceived breath freshening of the product. Cooling agents include, for example, menthol, ethyl *p*-menthane carboxamide, *N*-2,3-trimethyl-2-isoprylbutanamide, menthyl glutarate, menthyl succinate, menthol PC carbonate, menthol EC carbonate, menthyl lactate, menthone glyceryl ketal, menthol glyceryl ether, *N*-tertbutyl-*p-*menthane-3-carboxamide, *p*-menthane-3-carboxylic acid glycerol ester, methyl-2-isopryl-bicycle (2.2.1), heptane-2-carboxamide, menthol methyl ether and combinations thereof.

It is to be noted that the chewing gum may optionally contain a dental abrasive. Dental abrasives are particularly valuable in chewing gums because of the polishing action which occurs during chewing. The term "dental abrasives" as used herein includes all manner and form of such materials which are normally used in toothpastes, chewing gums and the like. Among the exemplary dental abrasives that may be used in the chewing gum are dicalcium diphosphate dihydrate, which also serves as an alkaline buffer, calcium carbonate, sodium metaphosphate, aluminum hydroxide, magnesium carbonate, calcium sulphate, silicas such as aerogels and xerogels, and tricalcium phosphate. The dental abrasive may typically be used in an amount of from about 1 to about 30 weight percent, or from about 2 to about 20 weight percent, based on the total weight of the chewing gum.

The chewing gum of the present invention may also optionally include other breath freshening, anti-microbial or oral health ingredients, such as for example non-toxic metallic salts selected from zinc and copper salts of gluconic acid, zinc and copper salts of lactic acid, zinc and copper salts of acetic acid, zinc and copper salts of citric acid and combinations thereof. Anti-microbial essential oils and flavor components, such as peppermint, methyl salicylate, thymol, eucalyptol, cinnamic aldehyde, polyphosphate, pyrophosphate and combinations thereof, may also be used. Finally, other dental health ingredients, in addition to those already detailed elsewhere herein, may be used, including for example proteolytic enzymes, lipids, anti-microbials, electrolytes, protein additives and combinations thereof.

In general, the chewing gum of the present invention may be prepared using any technique known in the art. In one embodiment, the chewing gum is prepared in tablet form, using compressed tableting techniques known in the art. In an alternative embodiment, the chewing gum is extruded, preferably in two or more layers using means known in the art (see, e.g., U.S. Pat. No. 6,783,783 which is incorporated herein by reference for all relevant purposes), one layer containing the calcium phosphate salts, and another layer containing a combination of acids, as set forth herein. In an embodiment of the present invention, the chewing gum is extruded in three-layers, the chewing gum comprising a center layer containing a combination of acids, and two outer layers containing the calcium phosphate salt, or a calcium salt and a phosphate salt, which may optionally act to essentially surround or encapsulate the center, acid-containing layer (such as in the case of, for example, a rope-like product).

The principles for extruding chewing gum are well known, and may be employed using known techniques in the art. In general, such a process involves the preparation of, in this instance, two or three mixtures containing the molten chewing gum base, along with all of the other chewing gum components or additives, with the exception that one or two of the molten mixtures comprises the calcium phosphate salts, and one of the molten mixtures comprises the combination of acids. Each of the mixtures is then simultaneously extruded through appropriately sized, shaped and positioned die or nozzles. After cooling sufficiently, the extrudate may then be gently formed into appropriately sized and shaped pieces.

### 3. Method of Mineralization or Remineralization

In general, mineralization or remineralization of a tooth surface may be accomplished by administering the oral composition of the present invention using conditions and techniques known in the art. It is desirable for the duration of the chewing gum in the oral cavity, as well as the rate at which the calcium phosphate salts, is released from the oral composition to be controlled so as to optimize the effectiveness of the product in mineralizing or remineralizing the tooth surface. For example, in the case of a chewing gum, administration typically comprises chewing the gum for at least about 1 minute, at least about 5 minutes, more typically for about 5 to about 60 minutes, even more typically for about 10 to about 30 minutes and, still more typically, for about 20 minutes. Furthermore, also in the case of a chewing gum for example, typically at least about 5 weight percent, about 10 weight percent, about 25 weight percent, about 50 weight percent, about 75 weight percent, or even about 100 weight percent of the calcium phosphate salts, is released from the gum during the first several minutes (e.g., the first about 2 minutes, about 3 minutes, about 4 minutes, about 5 minutes, about 10 minutes, or even about 20 minutes) of chewing. For example, in one embodiment, at least about 50 weight percent, about 75 weight percent, or even about 100 weight percent of the calcium phosphate salts, is released from the gum during the first about 20 minutes of chewing.

It is to be noted in this regard, however, that in various alternative embodiments, a more sustained delivery of active composition into the oral cavity may be desired. Thus, in such embodiments it may be desired for no more than about 25 weight percent, no more than about 50 weight percent, or no more than about 75 weight percent of the calcium phosphate salts, to release into the oral cavity during the first several minutes (e.g., the first about 2 minutes, about 3 minutes, about 4 minutes, about 5 minutes, about 10 minutes, or even about 20 minutes) of chewing. For example, in such an embodiment, it may be desired for no more than about 50 weight percent, or no more than about 75 weight percent, of the calcium phosphate salts, to release into the oral cavity during the first 20 minutes of administration.

Accordingly, in the case of, for example, a chewing gum containing a calcium phosphate salt and an acid blend, as set forth herein, generally a subject is encouraged to chew the gum for a certain period of time (e.g., at least about 5 minutes, at least about 10 minutes, at least about 20 minutes or more).

The present invention is further illustrated by the following Examples. These Examples are not to be regarded as limiting the scope of the invention or the manner in which it may be practiced.

### EXAMPLES

### Examples 1-17:

The following Tables detail exemplary chewing gum, candy, (as reference, not part of the invention) and compressed tablet compositions (as reference, not part of the invention) containing a combination of acids and a calcium phosphate salt, in accordance with the present invention.

**Table 1**

| **Chewing Gum Formulas** | | | | | | | |
|---|---|---|---|---|---|---|---|
| **(Wt.%)** | | | | | | | |
| Ingredient | Ex. 1 | Ex. 2 | Ex. 3 | Ex. 4 | Ex. 5 | Ex. 6 | Ex. 7 |
| Gum Base | 29.00 | 25.00 | 32.0 | 25.2 | 35.0 | 49.0 | 33.0 |
| Sorbitol | 35.76 | 35.76 | | 48.0 | 24.0 | 31.92 | 33.0 |
| Xylitol | 15.80 | 15.80 | | | 24.5 | | 7.0 |
| Liquid Sorbitol | | | | 6.5 | | 1.0 | |
| Sucrose | | | 55.4 | | | | |
| High Intensity Sweetener | 0.80 | 0.80 | | | | | |
| Aspartame | | | | | 0.5 | 0.10 | 0.30 |
| Encapsulated Acesulfame K | | | | 1.0 | 1.2 | | 3.0 |
| Sucralose | | | 0.10 | | | 0.50 | 0.05 |
| Corn Syrup | | | 2.0 | 1.0 | | | |
| Glycerin | 4.50 | 4.50 | | | 4.5 | 0.75 | |
| Lecithin | 0.09 | 0.09 | | | | 1.1 | 0.5 |
| Calcium Carbonate | | | 1.0 | 14.1 | | | 3.5 |
| Citric Acid | 2.00 | 2.00 | 2.5 | | 0.5 | 1.8 | |
| Encapsulated Malic Acid | | | | 1.0 | 0.5 | | 5.0 |
| Fumaric Acid | 1.00 | 1.00 | 1.0 | | 0.5 | 3.4 | 2.2 |
| Encapsulated Tartaric Acid | | | | 1.0 | 0.5 | | |
| Tricalcium Phosphate | | | 5.0 | 0.1 | | | 1.5 |
| Calcium Lactate | | | | | 5.3 | | 1.5 |
| TTCP/DCPA Blend * | 5.00 | 9.0 | | | 2.5 | | |
| Encapsulated Dicalcium Phosphate | | | | 0.1 | | 4.2 | 5.0 |
| Magnesium Stearate | 1.50 | 1.50 | | | | | |

| Ingredient | Ex. 1 | Ex. 2 | Ex. 3 | Ex. 4 | Ex. 5 | Ex. 6 | Ex. 7 |
|---|---|---|---|---|---|---|---|
| Sodium Citrate | 1.50 | 1.50 | | | | | |
| Sodium Metaphosphate | | | | | 0.5 | 1.0 | |
| Orange Flavor | | | 1.0 | 1.0 | 0.25 | | 4.39 |
| Eucalyptus Flavor | | | | 1.0 | | | 0.025 |
| Spearmint Flavor | | | | | 2.5 | 5.2 | |
| Other Flavor | 2.80 | 2.80 | | | | | |
| Color | 0.25 | 0.25 | | | | 0.025 | 0.010 |
| Menthol | | | | 1.0 | 0.25 | | 0.025 |
| TOTAL | 100.0% | 100.0% | 100.0% | 100.0% | 100.0% | 100.0% | 100.0% |

**Table 2**

| **Candy Formulas** | | | | | |
|---|---|---|---|---|---|
| **(Wt.%)** | | | | | |
| (as reference, not part of the invention) | | | | | |
| Ingredient | Ex. 8 | Ex. 9 | Ex. 10 | Ex. 11 | Ex. 12 |
| Corn Syrup | 43.01 | 38.00 | | | 45.50 |
| Sugar | 53.49 | 47.50 | | | 45.50 |
| Polyalcohols | | | 80.00 | 85.80 | |
| High Intensity Sweetener | | | 0.20 | 0.20 | |
| Malic Acid | 0.50 | 2.50 | | 0.50 | 1.00 |
| Citric Acid | | | 5.00 | 3.50 | 0.20 |
| Succinic Acid | 0.50 | 1.00 | 1.00 | 2.00 | 5.00 |
| Tetracalcium Phosphate | 0.50 | 5.00 | | 3.10 | |
| TTCP/DCPA Blend * | | | 10.0 | | |
| Dicalcium Phosphate | | | | 2.50 | |
| Sodium Pyrophosphate | 0.50 | | | 0.10 | |
| Color | 0.50 | 1.00 | 0.80 | 0.80 | 0.30 |
| Flavor | 1.00 | 5.00 | 3.00 | 1.50 | 2.50 |
| Total | 100.00% | 100.00% | 100.00% | 100.00% | 100.00% |

**Table 3**

| **Compressed Tablet Formulas** | | | | | |
|---|---|---|---|---|---|
| **(Wt.%)** | | | | | |
| (as reference, not part of the invention) | | | | | |
| Ingredient | Ex. 13 | Ex. 14 | Ex. 15 | Ex. 16 | Ex. 17 |
| Sorbitol | 96.35 | | 93.30 | 63.14 | 92.69 |
| Dextrose | | 94.15 | | | |
| Xylitol | | | | 27.31 | |
| Flavor | 1.0 | 1.0 | 1.0 | 1.0 | 1.0 |
| Magnesium Stearate | 0.10 | 0.10 | 0.10 | 0.10 | 0.10 |
| High Intensity Sweetener | 0.20 | 0.25 | 0.50 | 0.80 | 0.35 |
| Encapsulated Malic Acid | 0.25 | 1.50 | | 0.50 | 0.85 |
| Tartaric Acid | | | 3.00 | 1.50 | 0.10 |
| Fumaric Acid | 0.25 | 0.50 | 1.00 | 1.00 | 3.00 |
| Tetracalcium Phosphate | 0.50 | | 0.10 | | 0.21 |
| TTCP/DCPA Blend * | | | | 3.5 | |
| Dicalcium Phosphate | 0.25 | 1.00 | | | 0.50 |
| Sodium Hexametaphosphate | 0.10 | | | | 0.20 |
| Color | | 0.50 | | 0.15 | |
| Flavor | 1.00 | 1.00 | 1.00 | 1.00 | 1.00 |
| Total | 100.00 | 100.00 | 100.00 | 100.00 | 100.00 |

were encapsulated. The compositional differences, and the %release of calcium therefrom, are noted in the Table set forth below.

**Table 4**

| **Tab Gum, Mg Stearate-Coated TTCP/DCPA Salt Blend** | | |
|---|---|---|
| Acid Type | Wt% Acid per Tab | % Calcium Release |
| Citric (encap.) | 2.36 (2.0 encap.) | 34.0 |
| Citric (encap.), | 2.36 (2.0 encap.), | 46.1 |
| Fumaric | 1.0 | |
| Citric, | 2.0, | 57.7 |
| Fumaric, | 1.0, | |
| Sodium Citrate | 1.5 | |
| | | |

| **Tab Gum, Non-coated TTCP/DCPA Salt Blend** | | |
|---|---|---|
| Citric, | 2.0, | 77.4 |
| Fumaric, | 1.0, | |
| Sodium Citrate | 1.5 | |

In comparing the first two gum compositions (i.e., the composition containing encapsulated citric acid versus the composition containing fumaric acid with encapsulated citric acid), it is to be noted that the addition of fumaric acid helped to increase the percentage of calcium released. In comparing the second and third compositions (i.e., the composition containing fumaric acid with encapsulated citric acid versus the composition containing a blend of non-encapsulated citric and fumaric acids buffered with sodium citrate), it is to be noted that the addition of a buffer and/or the use of non-encapsulated citric acid resulted in yet a further increase in the percentage of calcium released. Finally, in comparing the third and fourth compositions, which differed only in that the fourth composition did not contain a TTCP/DCPA salt blend coated with magnesium stearate, it is to be noted that the latter composition released substantially more calcium.

## Claims

1. A chewing gum composition comprising a calcium phosphate salt, a first acid having a solubility of at least 25 weight percent in water at 20 °C and a second acid having a solubility of less than 20 weight percent in water at room temperature, wherein the weight ratio of the said first acid to the second acid is greater than 1:1, and wherein the calcium phosphate salt is a combination of tetracalcium phosphate and anhydrous dicalcium phosphate of from 1:1 to 5:1.

2. The chewing gum composition of claim 1, wherein said composition comprises 0.5 percent to less than 10 percent of the calcium phosphate salt combination, based on the total weight of the composition.

3. The chewing gum composition of any one of the preceding claims, wherein said first acid is selected from the group consisting of citric acid, succinic acid, tannic acid, lactic acid, ascorbic acid, gluconic acid, acetic acid, malic acid, tartaric acid and salts thereof.

4. The chewing gum composition of any one of the preceding claims, wherein said second acid is selected from the group consisting of adipic acid, fumaric acid, polyglutamic acid, polyaspartic acid, propionic acid, alginic acid, stearic acid, glutamic acid, aspartic acid, fatty acids, and salts thereof.

5. The chewing gum composition of any one of the preceding claims, wherein said composition has a total acid content of greater than 0.5 weight percent and less than 5 weight percent, based on the total weight of the composition.

6. A chewing gum composition as claimed in any preceding claim wherein
said first acid is selected from the group consisting of citric acid, succinic acid, tannic acid, lactic acid, ascorbic acid, gluconic acid, acetic acid, malic acid, tartaric acid and salts thereof;
said second acid is selected from the group consisting of adipic acid, fumaric acid, polyglutamic acid, polyaspartic acid, propionic acid, alginic acid, stearic acid, glutamic acid, aspartic acid, fatty acids, and salts thereof.
wherein said composition has a total acid content of greater than 0.5 weight percent and less than 5 weight percent, based on the total weight of the composition and wherein said composition comprises 0.5 percent to less than 10 percent of tetracalcium phosphate and anhydrous dicalcium phosphate based on the total weight of the composition.

7. The chewing gum composition of any one of the preceding claims, wherein the first acid is citric acid, and the second acid is fumaric acid.

8. The chewing gum composition of any preceding claim, wherein a portion of or substantially all of one or more of the acids is encapsulated.

## Patentansprüche

1. Kaugummizusammensetzung, die ein Calciumphosphatsalz, eine erste Säure mit einer Löslichkeit von mindestens 25 Gewichtsprozent in Wasser bei 20 °C und eine zweite Säure mit einer Löslichkeit von weniger als 20 Gewichtsprozent in Wasser bei Raumtemperatur umfasst, wobei das Gewichtsverhältnis der ersten Säure zu der zweiten Säure größer als 1:1 ist, und wobei das Calciumphosphatsalz eine Kombination von Tetracalciumphosphat und wasserfreiem Dicalciumphosphat von 1:1 bis 5:1 darstellt.

2. Kaugummizusammensetzung nach Anspruch 1, wobei die Zusammensetzung 0,5 Prozent bis weniger als 10 Prozent der Calciumphosphatsalzkombination umfasst, bezogen auf das Gesamtgewicht der Zusammensetzung.

3. Kaugummizusammensetzung nach einem der vorherigen Ansprüche, wobei die erste Säure ausgewählt ist aus der Gruppe, bestehend aus Citronensäure, Bernsteinsäure, Tanninsäure, Milchsäure, Ascorbinsäure, Gluconsäure, Essigsäure, Äpfelsäure, Weinsäure und deren Salzen.

4. Kaugummizusammensetzung nach einem der vorherigen Ansprüche, wobei die zweite Säure ausgewählt ist aus der Gruppe, bestehend aus Adipinsäure, Fumarsäure, Polyglutaminsäure, Polyasparaginsäure, Propionsäure, Alginsäure, Stearinsäure, Glutaminsäure, Asparaginsäure, Fettsäuren und deren Salzen.

5. Kaugummizusammensetzung nach einem der vorherigen Ansprüche, wobei die Zusammensetzung einen Gesamtsäuregehalt von mehr als 0,5 Gewichtsprozent und weniger als 5 Gewichtsprozent aufweist, bezogen auf das Gesamtgewicht der Zusammensetzung.

6. Kaugummizusammensetzung nach einem der vorherigen Ansprüche, wobei die erste Säure ausgewählt ist aus der Gruppe, bestehend aus Citronensäure, Bernsteinsäure, Tanninsäure, Milchsäure, Ascorbinsäure, Gluconsäure, Essigsäure, Äpfelsäure, Weinsäure und deren Salzen;
die zweite Säure ausgewählt ist aus der Gruppe, bestehend aus Adipinsäure, Fumarsäure, Polyglutaminsäure, Polyasparaginsäure, Propionsäure, Alginsäure, Stearinsäure, Glutaminsäure, Asparaginsäure, Fettsäuren und deren Salzen;
wobei die Zusammensetzung einen Gesamtsäuregehalt von mehr als 0,5 Gewichtsprozent und weniger als 5 Gewichtsprozent aufweist, bezogen auf das Gesamtgewicht der Zusammensetzung, und wobei die Zusammensetzung 0,5 Prozent bis weniger als 10 Prozent des Tetracalciumphosphats und wasserfreien Dicalciumphosphats umfasst, bezogen auf das Gesamtgewicht der Zusammensetzung.

7. Kaugummizusammensetzung nach einem der vorherigen Ansprüche, wobei die erste Säure Zitronensäure ist und die zweite Säure Fumarsäure ist.

8. Kaugummizusammensetzung nach einem der vorherigen Ansprüche, wobei ein Teil oder im Wesentlichen sämtliche der ein oder mehreren Säuren verkapselt sind.

## Revendications

1. Composition de pâte à mâcher comprenant un sel de phosphate de calcium, un premier acide ayant une solubilité d'au moins 25 pour cent en poids dans de l'eau à 20 °C et un second acide ayant une solubilité de moins de 20 pour cent en poids dans de l'eau à température ambiante, dans laquelle le rapport pondéral dudit premier acide audit second acide est supérieur à 1:1, et dans laquelle le sel de phosphate de calcium est une combinaison de phosphate tétracalcique et de phosphate dicalcique anhydre dans le rapport de 1:1 à 5:1.

2. Composition de pâte à mâcher selon la revendication 1, dans laquelle ladite composition comprend une combinaison de 0,5 pour cent à moins de 10 pour cent du sel de phosphate de calcium, sur la base du poids total de la composition.

3. Composition de pâte à mâcher selon l'une quelconque des revendications précédentes, dans laquelle ledit premier acide est choisi dans le groupe constitué de l'acide citrique, de l'acide succinique, de l'acide tannique, de l'acide lactique, de l'acide ascorbique, de l'acide gluconique, de l'acide acétique, de l'acide malique, de l'acide tartrique et de leurs sels.

4. Composition de pâte à mâcher selon l'une quelconque des revendications précédentes, dans laquelle ledit second acide est choisi dans le groupe constitué de l'acide adipique, de l'acide fumarique, de l'acide polyglutamique, de l'acide polyaspartique, de l'acide propionique, de l'acide alginique, de l'acide stéarique, de l'acide glutamique, de l'acide aspartique, des acides gras et de leurs sels.

5. Composition de pâte à mâcher selon l'une quelconque des revendications précédentes, dans laquelle ladite composition a une teneur totale en acides de plus de 0,5 pour cent en poids et de moins de 5 pour cent en poids sur la base du poids total de la composition.

6. Composition de pâte à mâcher selon l'une quelconque des revendications précédentes, dans laquelle
ledit premier acide est choisi dans le groupe constitué de l'acide citrique, de l'acide succinique, de l'acide tannique, de l'acide lactique, de l'acide ascorbique, de l'acide gluconique, de l'acide acétique, de l'acide malique, de l'acide tartrique et de leurs sels ;
ledit second acide est choisi dans le groupe constitué de l'acide adipique, de l'acide fumarique, de l'acide polyglutamique, de l'acide polyaspartique, de l'acide propionique, de l'acide alginique, de l'acide stéarique, de l'acide glutamique, de l'acide aspartique, des acides gras et de leurs sels ;
dans laquelle ladite composition a une teneur totale en acides de plus de 0,5 pour cent en poids et de moins de 5 pour cent en poids, sur la base du poids total de la composition et dans laquelle ladite composition comprend 0,5 pour cent à moins de 10 pour cent de phosphate tétracalcique et de phosphate dicalcique anhydre sur la base du poids total de la composition.

7. Composition de pâte à mâcher selon l'une quelconque des revendications précédentes, dans laquelle le premier acide est l'acide citrique et le second acide est l'acide fumarique.

8. Composition de pâte à mâcher selon l'une quelconque des revendications précédentes, dans laquelle une partie ou sensiblement la totalité d'un ou plus des acides est encapsulée.
